(19) **Europäisches Patentamt / European Patent Office / Office européen des brevets**

(11) **EP 3 262 416 B1**

(12) **EUROPEAN PATENT SPECIFICATION**

(45) Date of publication and mention
of the grant of the patent:
**26.12.2018 Bulletin 2018/52**

(21) Application number: **16709979.5**

(22) Date of filing: **23.02.2016**

(51) Int Cl.:
**G01N 33/574** *(2006.01)*    **G06F 19/00** *(2018.01)*

(86) International application number:
**PCT/EP2016/053726**

(87) International publication number:
**WO 2016/135119 (01.09.2016 Gazette 2016/35)**

(54) **METHOD FOR THE DIAGNOSIS OF ENDOMETRIAL CARCINOMA**

VERFAHREN ZUR DIAGNOSE VON ENDOMETRIUMKARZINOMEN

MÉTHODE POUR LE DIAGNOSTIC DU CARCINOME DE L'ENDOMÈTRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.02.2015 IT UB20157151**

(43) Date of publication of application:
**03.01.2018 Bulletin 2018/01**

(73) Proprietor: **Hosmotic SRL**
**80069 Vico Equense (NA) (IT)**

(72) Inventors:
• **TROISI, Jacopo**
**84090 Montecorvino Pugliano (SA) (IT)**
• **SCALA, Giovanni**
**80069 Vico Equense (NA) (IT)**
• **CAMPIGLIA, Pietro**
**80131 Napoli (NA) (IT)**
• **ZULLO, Fulvio**
**80121 Napoli (NA) (IT)**
• **GUIDA, Maurizio**
**80127 Napoli (NA) (IT)**

(74) Representative: **Longoni, Alessandra**
**AL & Partners Srl**
**Via C. Colombo ang. Via Appiani**
**(Corte del Cotone)**
**20831 Seregno (MB) (IT)**

(56) References cited:
**WO-A1-2011/161186    JP-A- 2007 057 309
JP-A- 2007 147 459**

• **KARKKAINEN J ET AL: "Comparison of mass spectrometry and radioimmunoassay to measure medroxyprogesterone acetate in patients with endometrial cancer", EUROPEAN JOURNAL OF CANCER AND CLINICAL ONCOLOGY, PERGAMON PRESS LTD, vol. 26, no. 9, 1 January 1990 (1990-01-01), pages 975-977, XP026199897, ISSN: 0277-5379, DOI: 10.1016/0277-5379(90)90624-3 [retrieved on 1990-01-01]**
• **CAO Q JACKIE ET AL: "Distinctive gene expression profiles by cDNA microarrays in endometrioid and serous carcinomas of the endometrium", INTERNATIONAL JOURNAL OF GYNECOLOGICAL PATHOLOGY, vol. 23, no. 4, October 2004 (2004-10), pages 321-329, XP008177794, ISSN: 0277-1691**
• **PETER PODOLEC ET AL: "Direct silylation of Trypanosoma brucei metabolites in aqueous samples and their GC-MS/MS analysis", JOURNAL OF CHROMATOGRAPHY B: BIOMEDICAL SCIENCES & APPLICATIONS., vol. 967, 1 September 2014 (2014-09-01), pages 134-138, XP055220248, NL ISSN: 1570-0232, DOI: 10.1016/j.jchromb.2014.07.023**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a method for the diagnosis of endometrial carcinoma based on metabolomic analysis of blood and bioinformatics manipulation of metabolic profiles through classification models.

The endometrial carcinoma is the most common invasive cancer of the female genital tract and it is responsible of 7% of all invasive tumours in women (excluding cutaneous tumours).

The endometrial carcinoma is rare in women having less than 40 years. The peak of incidence is between 55 and 65 years. Clinical-pathological studies and molecular analysis have supported the classification of endometrial carcinoma into two broad categories: Type I and Type II.

The type I is the most frequent, with a percentage of cases higher than 80%, it mines the endometrial proliferative glands and it is so defined with the term endometrioid carcinoma. In general, it arises in a frame of endometrial hyperplasia and, like this one, it is associated with obesity, diabetes, hypertension, infertility and uncontested oestrogenic stimulation. Recent studies have provided further evidence supporting the thesis that endometrial hyperplasia is a precursor of endometrial carcinoma (Muller GL et al. Allelotype mapping of unstable microsatellites establishes direct lineage continuity between endometrial precancers and cancers. Cancers Res 56:4483, 1996). The type II endometrial carcinoma generally affects women ten years later than the type I endometrial carcinoma (65-75 years) and, differently from type I, it most of all develops on a frame of endometrial atrophy. The type II represents less than 15% of endometrial carcinoma cases and it is scarcely differentiated (G3). The most common subtype is the serous one, that is so defined due to the biological and morphological overlapping with the ovarian carcinoma. Less common histological subtypes also belong to this category: clear cell carcinoma and malignant mixed Mullerian tumour.

At the moment, a mass screening on an asymptomatic population in perimenopausal and postmenopausal age for the early diagnosis of endometrial carcinoma, as it is carried out for the cervical carcinoma through Pap-test, is not feasible.

Studies carried out on an exocervical sample have proven a frequency of false negatives of about 40-50% since the endometrial exfoliated cells, having undergone the action of the vaginal environment, present alterations and therefore lose the characteristics that allow the differentiation of the tumour cell from the normal cell. Moreover the prognosis is strictly bound to the earliness of the diagnosis, in fact the survival after 5 years drastically diminishes from 78-98% in case of diagnosis at stage I till 3-10% in case of diagnosis in stage IV.

To date, several thousands of metabolites of the human serum have been identified and the application of metabolomics has allowed the development of biomarkers for many diseases such as schizophrenia (Kaddurah-Daouk R., Metabolic profiling of patients with schizophrenia, PLOS Med 2006; 8:e363), meningitis (Subramanian A. et al., Proton MR/CSF analysis and a new software as predictors for the differentiation of meningitis in children, NMR Biomed 2005; 18:213-25) and colon cancer (Denkert C., et al., Metabolite profiling of human colon carcinoma - deregulation of TCA cycle and amino acid turnover, Mol. Cancer 2008; 7:1-15). Nevertheless the use of metabolomics in gynaecological field has been till now limited to studies concerning ovarian carcinoma (Fan L. et al. Identification of metabolic biomarkers to diagnose epithelial ovarian cancer using a UPLC/QTOF/MS platform Acta Oncologica, 2012; 51:473-479). To date, there are no studies reported in literature carried out in gascromatography coupled to mass spectrometry and with chemiometric techniques for the diagnosis of the endometrial carcinoma.

It is therefore strongly needed a non-invasive diagnostic system which allows to carry out a screening on the population at risk for age or for known risk factors, in order to early identify this fearful female neoplasia.

Advantageously, the present invention solves the above mentioned problems through a non-invasive method for the diagnosis of endometrial carcinoma. Up today, there are no other non-invasive diagnostic methods which allow such a histological distinction of this kind of tumour.

The object of the invention will be hereinafter explained in detail.

BRIEF DESCRIPTION OF THE FIGURES

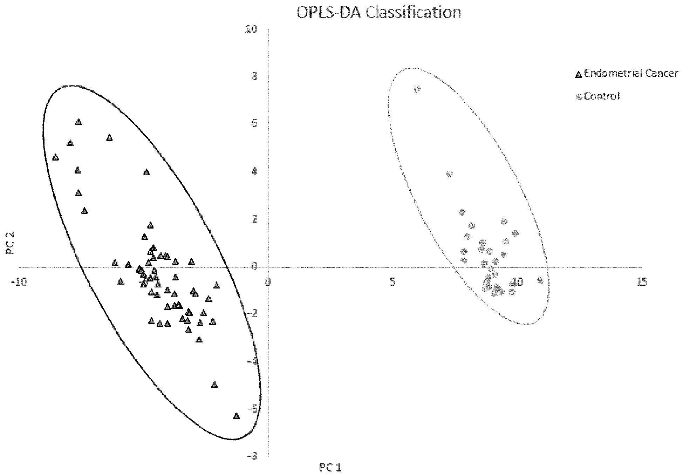

Figure 1 shows the result of the analysis OPLS-DA based on data of the metabolomic profile of the patients with endometrial carcinoma and of healthy controls.

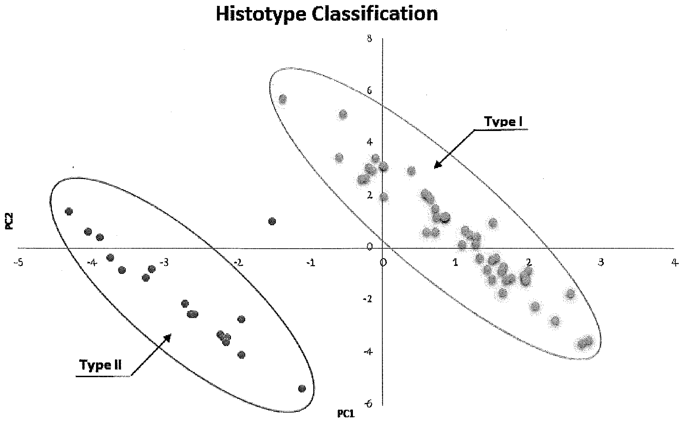

The scores plots discriminate between the two classes without overlappings. The triangles represent the patients affected by endometrial carcinoma, whereas the small rings the healthy patients. The main components PC1 and PC2 reported on the axes respectively disclose the 16.5% and the 14.9% of the global variance. Figure 2 shows, according to the invention, the histological classification (carcinoma of type I vs carcinoma of type II) obtained with the PLS-DA model. The spots represent the metabolomic profiles of women with endometrial carcinoma of type I, whereas the triangles the ones of the patients with endometrial carcinoma of type II. Only one of these samples is placed by the model in an area which is not univocally attributable to the correct area.

DEFINITIONS

With the term "metabolomics", the analysis of cellular processes by the metabolomics profile study of small molecules of an organism is intended.

With the term "metabolomic analysis" the inventors wish to refer to the carrying out of a process aimed at the identification and the determination of the concentration of the greatest possible number of metabolites in a biological sample.

With the term "metabolites" the small molecules derived from the biological processes of anabolic or catabolic type of a cell or of a set of cells are intended. With the term "metabolites" the inventors wish to refer to all the molecules having a molecular weight lower than 1000 Dalton, which are potentially identifiable and measurable within a biological sample.

With the term "metabolomic profile" the specific pattern that the metabolites have in the blood of the patient depending on their relative proportions is intended.

The PLS-DA (Partial Least Squares Discriminant Analysis) is a supervised method which uses techniques of multivariate regression to extract through linear combinations of the original variables (X) the information that may predict the pertinence to a determinate class (Y). In order to evaluate the effectiveness in discrimination of the classes, a permutation test is performed. In each permutation, a PLS-DA model is built from the data (X) and the commuted class labels (Y) by using the optimal numbers of components determined by cross validation for the model based on the assignment of the original classes. Two types of statistical tests are performed to measure the discrimination power between the classes. The first one is based on the prediction accuracy in the training phase of the model. The second one is based on the separation distance according to the ratio between the sum of the quadratic distances within the classes and among the classes (B/W-ratio).

The OPLS-DA (Orthogonal Partial Least Squares - Discriminant Analysis) is an important development of the technique PLS-DA that has been proposed to orthogonally manage the variation of the classes in the data matrix.

OPLS-DA increases the classification performances of the models PLS-DA. The performances of classification are estimated on the basis of "k-fold cross validation" by dividing the data matrix in k random subsets. For each calculation cycle, one of the subsets of F is kept aside as a test set and the remaining k-1 subsets act as trainers. Each of the K subsets is used one time as a test set, generating K precision values. The accuracy of the classification is calculated as the average of the accuracy rates in k subsets. The model is subjected to cross validation with the method "leave one out cross validation" (LOOCV) in order to be validated. The data matrix is scaled to the mean and the unit variance, before being submitted to the division into k subsets. In other words, the average and the standard deviation of the training data are used to indicate the center and to scale the test data. Once trained, the model is used to check whether the data have generated an "overfitting". To do this, a validation set with known class labels is created and it is thus checked whether it gives an accuracy rate comparable to that of the training data. Another method is a plot validation $R^2/Q^2$ which helps to assess the risk that the current model is spurious, that is, the model fits well only to subsets set but does not predict Y just as well for the new observations. The value of $R^2$ is the percentage variation of the training set that can be explained by the model.

The value of $Q^2$ is a cross-validated measure of $R^2$. This validation compares the goodness of fit of the original model with the goodness of fit of different models based on the data in which the order of observations Y is permuted randomly, while the matrix is kept intact. The criteria for the validity of the model are the following:

1. All the $Q^2$ values on the permuted data set must be lower than the $Q^2$ value, estimated on the current data set. If this is not checked, it means that the model is overfitted.
2. The regression line (the line joining the actual point $Q^2$ to the centroid of the cluster of $Q^2$ permuted values) has a negative value of the y-axis intercept.

Support Vector Machines (SVMs) are machine learning supervised techniques relatively new for classification uses. The SVMs were proposed for the first time in 1982 by Vapnik (Vapnik, V. Estimation of Dependences Based on Empirical Data; Springer Verlag: New York, 1982). The basic principle of SVMs, which are essentially binary classifiers is the following: given a set data with two classes, a linear classifier is constructed in the form of a hyperplane, which has the maximum margin in the simultaneous minimization of the empirical classification error and the maximization of the geometric margin. In the case of data sets that are not linearly separable, the original data are mapped into a higher dimensional feature space and a linear classifier is built in this new space (this is known as the "kernel"). Considering a set of training data $x_i \in \mathbb{R}n$, $i = 1,...,m$ where each of $x_i$ falls into one of the two categories $y_i \in \{1,1\}$, SVM determines the hyperplane whose parameters are given by (w,b) as obtained by the solution of the following convex optimization problem:

$$\min_{w,b,\varepsilon} \frac{1}{2} w^t w + c \sum_{i=1}^{m} \varepsilon_i$$

subjected to the following conditions:

$$y_i(w^t x_i + b) \geq 1 - \varepsilon_i$$

$$\varepsilon_i \geq 0$$

wherein c is the regularization parameter, which is a compromise between the learning accuracy and the term prediction, and $\varepsilon$ is a measure of the number of classification errors. The inclusion of the term regularization reduces the problem of overfitting.

DECISION TREES.

Decision trees build classification models based on recursive partitioning of data. Typically, an algorithm of the decision tree begins with the entire set of data, the data are divided into two or more subgroups based on the values of one or more attributes, and then each subset is repeatedly divided into smaller subsets until the size of each subset reaches an appropriate level. The entire modeling process can be represented in a tree structure, and the generated model can be summarized as a set of rules *"if-then"*. Decision trees are easy to interpret, computationally undemanding, and able to cope with noisy data. Most of the decision trees tackles the classification problems, such as for example the object of this invention. In this context, the technique is also referred to as classification tree. In the representation with the tree structure, a knot represents a set of data, and the entire set of data is represented as a knot at the root.

JP2007057309 discloses a computer assisted method for the diagnosis of endometrial carcinoma based on a proteomic analysis of blood.

KARKKAINEN J ET AL, EUROPEAN JOURNAL OF CANCER AND CLINICAL ONCOLOGY, vol. 26, no. 9, 1990, pages 975-977 discloses a GC-MS analysis of administered medicaments (e.g. MPA) in the blood of endometrial carcinoma patients for the monitoring of the treatment.

WO 2011/161186 discloses diagnosis of sepsis and mentions a variety of specific type of multivariate analysis or classification algorithm methods.

CAO Q JACKIE ET AL, International Journal of Gynecological Pathology 23(4), pages 321-329 discloses gene expression profiles by cDNA microarrays in endometrioid and serous carcinomas of the endometrium.

JP 2007 147459 discloses ribitol as an internal standrad.

PETER PODOLEC ET AL, JOURNAL OF CHROMATOGRAPHY B: BIOMEDICAL SCIENCES & APPLICATIONS., vol. 967, 2014, pages 134-138, discloses GC-MS/MS analysis of Trypanosoma brucei metabolites.

DETAILED DESCRIPTION OF THE INVENTION

The present invention relates to a computer assisted method for the diagnosis of endometrial carcinoma, based on metabolomic analysis of blood and on an integration of the obtained results through a multivariate analysis using models of discriminant analysis selected in the group consisting of PLS-DA and OPLS-DA, or models of computer learning selected in the group consisting of SVM and decision tree.

The object of the present invention is a method for the diagnosis of the endometrial carcinoma based on metabolomic analysis of blood, said method comprising the following phases:

    (I) a training phase comprising:

       - GCMS or GCxGCMS analysis of blood samples derived from patients with endometrial carcinoma and healthy controls;
       - integration of the obtained results by multivariate analysis using at least a discriminant analysis model or a model of *computer learning* to train at least a classification model;

    (II) an assignment phase comprising GCMS or GCxGCMS analysis of an unknown blood sample and its assignment to a class on the basis of the classification model formulated in the training phase (I).

The multivariate analysis, carried out on collected chromatograms using:

- at least a discriminant analysis model selected from the group consisting of: PLS-DA and OPLS-DA, or
- said model of computer learning selected from the group consisting of: SVM and *decision tree*;

has advantageously allowed the satisfactory dichotomous classification ("Healthy Patient" vs "Patient affected by endometrial carcinoma") of unknown samples. The classification model obtained with a multivariate PLS-DA analysis has even allowed the histological discrimination of the carcinoma (carcinoma of type I vs carcinoma of type II). To date, there are no other non-invasive diagnostic methods which may allow such a histological discrimination of this kind of tumour.

In said training phase (I) the samples derived from patients affected by endometrial carcinoma and from healthy women with similar physical (BMI, age, co-morbidity) and social (level of education, socio-economic condition) characteristics are analysed, and in this way the classification models are trained. This training phase is aimed at creating and delimiting the characteristics of the metabolic profile present in the blood of the two groups. In order to have a good predictivity of the classification model it is necessary to subject to a multivariate analysis a number of blood samples derived from patients with endometrial carcinoma and from healthy controls equal to at least 80% of the number of the identified variables of metabolic profiles, such samples belonging to at least 2 different classes.

In such assignment phase (II) the unknown samples are subjected to GCMS analysis, and the resulting chromatograms are classified according to the previously trained models, estimating the most probable class of pertinence.

The method of diagnosis of the endometrial carcinoma of the present invention is not based on the measurement of the concentration of each metabolite, but the whole cluster of metabolites is considered as biomarker (metabolic profile), which, for being present according to different proportions in the 2 groups, allow the insertion into two different classes of pertinence.

Preferably, said training phase (I) further comprises the following sub-phases:

- extraction and derivatization of metabolites from blood samples derived from patients with endometrial carcinoma and from healthy controls;
- GCMS or GCxGCMS analysis of metabolites extracted and derivatized to obtain a chromatogram for each sample, each chromatogram being a metabolic profile;
- data matrix creation of the metabolic profiles of patients with endometrial carcinoma and of healthy controls;
- structuring of at least a classification model as a result of data array multivariate analysis; wherein said multivariate analysis is carried out using at least a discriminant analysis model or a model of computer learning to train at least a classification model.

Different classification models can be used according to the present invention; preferably said classification models are selected from the group consisting of: PLS-DA, OPLS-DA, SVM and Decision Tree.

Preferably said assignment phase (II) further comprises the following sub-phases:

- extraction and derivatization of metabolites from at least an unknown blood sample;
- GCMS or GCxGCMS analysis of the metabolites extracted and derivatized to obtain at least a chromatogram for the unknown blood sample;
- metabolic profile creation from said chromatogram of the unknown blood sample;
- assignment of the metabolic profile to a class on the basis of the model of classification trained in phase (I).

Preferably, the method of the present invention envisages a classification model trained for a dichotomous classification "Healthy Patient" or "Patient affected by endometrial carcinoma". Even more preferably, said classification model is also trained for a histolological classification of "type I" or "type II" cancer.

Preferably, said extraction is carried out using an extraction mixture consisting of an aqueous mixture of an alcohol and of an aprotic polar solvent, preferably $CH_3OH/H_2O/CHCl_3$, even more preferably with a volume ratio 2-3/0.5-0.5/0.5-1.

In a preferred embodiment, said extraction and derivatization sub-phase comprises:

i) stirring of the sample obtained from addition of an extraction mixture;
ii) centrifugation of the sample obtained in i);
iii) derivatization of the supernatant obtained from ii) by treatment with methoxyamine hydrochloride in pyridine;
iv) supernatant silanization of the sample obtained in iii) with a silanization agent selected from the group consisting of: N,O-bis(trimethylsilyl) trifluoroacetamide (BSTFA), N-methyl-N-(trimethylsilyl) trifluoroacetamide (MSTFA), esamethyl disilazane (HMDS), 1-(trimethylsilyl) imidazole (TMSI), N-tert-butyldimethylsilyl-N-methyltrifluoroacetamide (MTBSTFA), 1-(tert-butyldimethylsilyl) imidazole (TBDMSIM) in the optional presence of trimethychlorosilane (TMCS).

Preferably, said extraction of metabolites is carried out after having added to the sample a known aliquot of a reference compound; preferably said reference compound is ribitol.

In order to obtain the separation of metabolites useful for the purposes of the present invention it is possible to work with both monodimensional gas chromatography and with two-dimensional gas chromatography; two-dimensional gas chromatography is preferred since the better resolving power of the technique offers a better classification accuracy. Anyway, as shown in the EXAMPLES it is also possible to work with the more common monodimensional gas chromatography.

The obtained gas chromatograms, preferably in SCAN mode, are integrated so as to identify all the peaks having an area greater than 10 times the background noise of the chromatogram trace.

Using the peak of the reference compound (preferably ribitol) as a reference both for the quantitative analysis and to center the retention times, each peak is identified on the basis of one signal m/z of quantization and at least 2 signals m/z of qualification. After the integration the quantification with the method of normalized percentages areas is carried out. The obtained results from this quantization (normalized percentages areas) are transferred to a matrix wherein each sample represents a line and the columns are represented by various metabolites univocally identified by means of their gas chromatographic retention time, compared to the retention time of the reference compound. The first column of the matrix is used to define the class of pertinence of the sample. In the easiest case only two classes can be envisaged "Healthy Patient" and "Patient affected by endometrial carcinoma", further on are reported evidences of the working of the invention on the basis of this dichotomous classification.

It is also object of the present invention a method as disclosed above further comprising the following phases:

- integration of chromatograms, wherein said integration provides for the identification of all peaks that have an area greater than 10 times the background noise of the chromatogram trace; using the peak of the reference compound as reference both for the quantitative analysis and to center the retention times,

where each peak is identified on the basis of:

- one signal m/z of quantization; and
- at least two signals m/z of qualification;
- quantification with the method of normalized percentages areas;
- transfer of the data obtained from said quantification to a matrix in which each sample represents a line and the columns are represented by various metabolites univocally identified by means of their chromatographic retention time.

The multivariate statistical analysis of data (PLS-DA and OPLS-DA) and the automatic learning (SVM and decision tree) are carried out on normalized and corrected chromatograms (based on the peak area of ribitol) using SIMPCA-P 13.0 (Umetrics), RapidMiner 5.3 (Rapid-I) and R (Foundation for Statistical Computing, Vienna). The values are centered on the average and the variance is normalized. For the metabolic profile, the model OPLS-DA has shown satisfactory ability of modelling and predictivity using a predictive component and three orthogonal components ($R^2Y_{cum}$=0.995, $Q^2_{cum}$=0.985). Figure 1 shows the separation between classes obtained with OPLS-DA model.

Moreover, a classification based on the histology of the carcinoma through a model PLS-DA was built. As shown in Figure 2, only one sample is placed in an uncertain area of the definition space of the classes.

The present invention can be better understood in the light of the following nonlimiting examples.

EXAMPLES

The diagnostic methodology object of the present invention was developed starting from metabolomic analysis, carried out on blood samples collected from patients with certain diagnosis of endometrial carcinoma, before the intervention of hysterectomy and from a group of control women having similar physical and socio-economic characteristics but with a healthy uterus. The information about the isotype and the neoplasia stage were collected after the hysterectomy on the basis of the anatomopathological evidences obtained by the analysis of the explanted organ.

Collection of samples

The samples were taken from 88 women with endometrial carcinoma and 80 healthy women, who voluntary gave samples of blood. The study was approved by the ethical committee of the university of Magna Grecia of Catanzaro and the patients and the healthy volunteers signed the informed consent about the purposes of the study. The samples of blood were taken just before the hysterectomy intervention using vials BD Vacutainer®, the serum was frozen at -80°C till the time of analysis. The diagnostic suspect of endometrial carcinoma after the hysterectoscopic test with biopsy of the endometrial lesion was confirmed by the anatomopathological test of the uterus after the hysterectomy intervention. A control group was also arranged taking blood samples from women having no signs of endometrial carcinoma and with similar physical and socio-economic characteristics (weight, height, BMI, age, civil status, level of education and so).

The demographic and clinical characteristics of the cases and of the controls are reported in Table 1 while in Table 2 the anatomopathological characteristics of the investigated tumours are listed.

Table1: characteristics of the population of the study

| Parameter | Endometrial carcinoma | Controls | P value |
|---|---|---|---|
| Number of cases | 88 | 80 | -- |
| Age (years) | $63.3 \pm 14.8$ | $63.1 \pm 8.3$ | NS |
| BMI | $27.6 \pm 6.7$ | $26.2 \pm 4.5$ | NS |

Table 2: anatomopathological characteristics of the investigated tumours

| | | Number of cases | Percentage of cases |
|---|---|---|---|
| Histotype | Tipo I | 67 | 76.1% |
| | Tipo II | 21 | 23.9% |
| Stage | G1 | 2 | 2.3% |
| | G2 | 53 | 60.2% |
| | G3 | 33 | 37.5% |

Extraction and derivatization of metabolites

Fifty microliters of serum were transferred into 2 mL Eppendorf vials and 20 $\mu$L of a 1 g/L solution of ribitol and 200 $\mu$L of a mixture consisting of 2.5 parts of methanol, 1 part of water and 1 part of chloroform ($CH_3OH:H_2O:CHCl_3$, 2.5:1:1) were added. The solution was mixed in vortex for 30 seconds.

The samples were then centrifuged at 16000 rpm for 10 minutes at 4°C. An aliquot of 200 $\mu$L of supernatant was collected and transferred in new 2 mL Eppendorf vials and added with 200 $\mu$L of $H_2O$ and mixed in vortex for 30 seconds and centrifuged again at 16000 rpm for 5 minutes at 4°C.

An aliquot of 350 $\mu$L of the supernatant was collected again and transferred into 1.5 glass ampoules and lyophilized.

The lyophilized sample was treated with 50 $\mu$L of 20 mg/mL methoxyamine hydrochloride in pyridine. The reaction was carried out at 37°C under stirring (350 rpm) for 90 minutes. At the end, 50 $\mu$L di N,O-bis(trimethyllsilyl)trifluoroacetamide (BSTFA) with 1% of trimethylchlorosilane were added to each ampoule and the silanization reaction was carried out at 37°C for 60 minutes under stirring (350 rpm).

MDGCMS analysis

For two dimensional gas chromatography a primary column (placed in the first oven) was used, of the type SLB-5ms 30.0 m x 0,25 mm ID with 1 $\mu$m of thickness of film [silphenylene polymer, practically having equivalent polarity to poly(5% diphenyl/95% methylsiloxane)] (J&W Agilent) which was bound to the position 1 of the interface with 7 doors (SGE).

A BPX-50 5,0 m x 0,50 mm ID with 0.25 $\mu$m of thickness of the film was bound to the position 7 of the interface. A BPX-50 1.5 m x 0.25 mm ID, 0.25 $\mu$m was set to position 6 and connected to a flame ionisation detector (FID) set at 320°C, while the analytical column of 5.0 m (chemically identical to the one connected to FID) was connected to system qMS.

The column connected to FID was used to reduce the flux in the second dimension and to check that the scarcely representative compound was not due to a random fluctuation of the chromatography.

It was used a 40 $\mu$L (20 cm x 0.71 mm OD x 0.51 mm ID in stainless-steel) outer capillary vessel to connect the doors 3 and 4 of the interface SGE.

The thermal program equal for the two ovens was: 80°C for 1 minute then heating till 320°C at 3°C/minute and maintained for 4 minutes.

The starting pressure of helium (constant linear velocity) was set at 129.6 kPa. The auxiliary starting pressure of helium of the APC (advanced control of pressure), which also works in constant linear velocity conditions was set at 90.4 kPa.

The injection volume of 1 $\mu$L with a split ratio of: 1:5. The modulation period was set at 4.1 s (accumulation period 4.0 seconds, injection period 0.1 seconds). The conditions of the quadrupole mass spectrometer were: ionization mode: electronic impact (70 eV), mass range:40-600 m/z, scanning rate: 10.000 amu/second.

GCMS analysis

For the monodimensional gas chromatography a column of the type CP-Sil 8 CB GC Column, 30 m, 0.25 mm, 1,00 $\mu$m, (Agilent J&W) was used.

The thermal program of GC envisaged a starting temperature of 100°C per 1 minute then heating till 320°C at 4°C/minute and 4 minutes of hold time for a total running time of 60 minutes.

The starting pressure of helium (constant linear velocity of 39 cm/s) was set at 83.7 kPa. The injecton volume at 2 $\mu$L with a split ratio: 1:5. The conditions of the quadrupole mass spectrometer were: ionization mode: electronic impact (70 eV), mass range: 35-600 m/z, scanning rate: 3.333 amu/second with a solvent cut time of 4.5 minutes.

Creation of the matrix data

In a TIC chromatogram are usually detected more than 250 signals, some of these peaks were not further investigated since there were no correspondences in other samples, because they were in too low concentration or because they had a poor spectral quality to be confirmed as metabolites.

A total of 198 endogenous metabolites such as amino acids, organic acids, carbohydrates, fatty acids and steroids were detected. For the identification of the peak, the linear retention index was used (LRI) setting as maximum tolerance a difference between the tabulated Kovats index and the experimental index of 10, while the minimum of compatibility for the search in the libraries was set at 85%. 2 libraries were used: the NIST11 and a library purposely developed by derivatizing more than 500 metabolites in the same conditions of the analysed samples. The areas of the peaks were normalized and corrected with reference to the signal of ribitol. The results were summarized in a matrix file separated by commas (CSV) and loaded in a suitable software for the statistical processing.

Gas chromatograms obtained in SCAN mode were integrated so as to identify all the peaks having an area greater than 10 times the background noise of the gas chromatogram trace. Each peak was identified on the basis of signal m/z of quantization and at least two signals m/z of qualification. After the integration, the quantification with the method of normalized percentages areas was carried out, the ribitol peak was used as reference both for quantitative analysis and to center the retention times.

The results obtained from this quantization (normalized percentages areas) were transferred to a matrix wherein each sample represent a line and the columns were represented by various metabolites univocally identified by means of their gas chromatographic retention time. The first column of the matrix is used to define the class of pertinence of the sample. In the easiest case only two classes can be envisaged "Healthy Patient" and "Patient affected by endometrial carcinoma", further on are reported evidences of the working of the invention on the basis of this dichotomous classification. Further evidences were obtained about the possibility of different classification models tested also to predict the histotype of the neoplasia and the grading.

Statistic analysis

The multivariate statistical analysis of data (PLS-DA and OPLS-DA) and the automatic learning (SVM and decision tree) were carried out on the normalized and corrected chromatograms (based on the peak area of ribitol) using SIMPCA-P 13.0 (Umetrics), RapidMiner 5.3 (Rapid-I) and R (Foundation for Statistial Computing, Vienna).

The values were centered on the average and the variance was normalized.

Results

For a metabolic profile, the model OPLS-DA has shown satisfactory ability of modelling and predictivity using a predictive component and three orthogonal components ($R^2Y_{cm}=0.995$, $Q^2_{cum}=0.985$). The other models of classification have shown good (even if lower than OPLS-DA) classification abilities. Different approaches are possible for the final assignment of the class of pertinence of the unknown sample. The answer of a sole model can be used or the answers of the various models can be integrated in a more complex decisional algorithm.

Table 3 reports some indexes of the assessment of diagnostic performances used to evaluate the investigated models. The sensitivity was calculated as TP/(TP+FN), wherein TP represents the number of true positives, namely correctly diagnosticated samples as affected by endometrial carcinoma by the proposed model, and FN is the number of false negatives, namely the samples erroneously identified as negatives. The specificity was calculated as TN/(TN+FP), wherein TN represents the number of true negatives, namely samples correctly diagnosticated as healthy and FP represents the false positives, namely the number of people erroneously diagnosticated as healthy. The ratio of positive likelihood (PLR) was calculated as Sensitivity/(1-Specificity), while the negative one (NLR) as (1-Sensitivity)/Specificity.

The predictive value (NPV) was calculated as TN/(TN+FN), while the positive (VPP) as TP/(TP+FP). The accuracy represents the percentage of all the correct assignments and was calculated as (TP+TN)/(TP+FP+TN+FN) while the repeatability as the numbers of correct reassignments in 10 replications of the analysis of a sample.

Table 3 - Diagnostic performance of the investigated models

| Parameter | OPLS-DA | PLS-DA | SVM | Decision tree |
|---|---|---|---|---|
| *Sensitivity* | | 0.989 | 0.966 | 0.977 |
| *Specificity* | | 0.988 | 0.974 | 0.963 |
| *PLR* | | 79.1 | 37.7 | 26.1 |
| *NLR* | No classification error | 0.012 | 0.035 | 0.024 |
| *NPV* | | 0.988 | 0.962 | 0.975 |
| *PPV* | | 0.989 | 0.977 | 0.966 |
| *Accuracy* | | 0.988 | 0.970 | 0.970 |
| *Repeatability* | | >99% | >99% | >99% |

In order to identify the metabolites that much more contributed to the separation of the classes, it was calculated the score of the important variables in the projection (VIP) for each component. VIP scores represent the weighted sum of the squares of loading of the pls, considering the amount of y-variance in any dimension. Two peaks show a VIP score greater than 2 in both the models PLS-DA and OPLS-DA (both in the classification of endometrial carcinoma vs control and in the classification of type I vs type II. These were identified as important knots also in the decision tree, these observations suggest a great importance of these variables in the classification processes (not reported data). The first metabolite (VIP-score=2,3; spectrometric similarity =91%; $\delta$LRI = 11) resulted to be a signal attributable to glutamine amino acid, while the second (VIP-score=2,1; spetrometric similarity=89% $\delta$LRI = 16) resulted to be attributable to glucono $\delta$-lactone.

**Claims**

1. A computer assisted method for the diagnosis of endometrial carcinoma based on metabolomic analysis of blood, said method comprising the following phases:

   (I) a training phase comprising:

   - GCMS or GCxGCMS analysis of blood samples derived from patients with endometrial carcinoma and healthy controls;
   - integration of the obtained results by a multivariate analysis using at least a discriminant analysis model or a model of computer learning to train at least a classification model;

   (II) an assignment phase comprising GCMS or GCxGCMS analysis of an unknown blood sample and its assignment to a class of pertinence on the basis of the classification model formulated in the training phase (I).

2. The method according to claim 1 wherein

- at least a discriminant analysis model is selected from the group consisting of: PLS-DA and OPLS-DA, or
- said model of computer learning is selected from the group consisting of: SVM and decision tree.

3. The method according to one or more of the previous claims, wherein the training phase (I) comprises the following sub-phases:

- extraction and derivatization of metabolites from blood samples derived from patients with carcinoma and from healthy controls;
- GCMS or GCxGCMS analysis of metabolites extracted and derivatized to obtain a chromatogram for each sample;
- data matrix creation of the metabolic profiles of the patients having endometrial carcinoma and of healthy controls;
- structuring of at least a classification model as a result of data array multivariate analysis; wherein said multivariate analysis is carried out using at least a discriminant analysis model or a model of computer learning to train at least a classification model.

4. The method according to one or more of the preceeding claims, wherein said phase (II) further comprises the following sub-phases:

- extraction and derivatization of metabolites from at least an unknown blood sample;
- GCMS or GCxGCMS analysis of the metabolites extracted and derivatized to obtain at least a chromatogram for the unknown blood sample;
- metabolic profile creation from said chromatogram of the unknown blood sample;
- assignment of the metabolic profile to a class on the basis of the classification model trained in phase (I).

5. The method according to one or more of the previous claims wherein the number of blood samples derived from patients with endometrial carcinoma and from healthy controls is equal to at least 80% of the number of identified variables of metabolic profiles.

6. The method according to one or more of the previous claims wherein said classification model is trained for a dichotomous classification "Healthy Patient" or "Patient affected by endometrial carcinoma".

7. The method according to one or more of the previous claims wherein said classification model is further trained for a histological classification of "type I" or "type II" cancer.

8. The method according to one or more of the previous claims wherein said extraction and derivatization comprise:

i) stirring of the sample obtained from the addition of an extraction mixture;
ii) centrifugation of the sample obtained in i);
iii) derivatization of the supernatant obtained in ii) by treatment with methoxyamine hydrochloride in pyridine;
iv) supernatant silanization of the sample obtained in iii) with a silanization agent selected from the group consisting of: N,O-bis(trimethylsilyl) trifluoroacetamide (BSTFA), N-methyl-N-(trimethylsilyl) trifluoroacetamide (MSTFA), hexamethyl disilazane (HMDS), 1-(trimethylsilyl) imidazole (TMSI), N-tert-butyldimethyllsylyil-N-methyiltrifluoroacetamide (MTBSTFA), 1-(tert-butyldimethylilsilyl)imidazole (TBDMSIM);

wherein said extraction mixture consists of an aqueous mixture of an alcohol and an aprotic polar solvent.

9. The method according to one or more of the previous claims wherein said extraction of metabolites is performed by adding an aliquot of a reference compound, preferably ribitol.

10. The method according to one or more of the previous claims further comprising the following phases:

- integration of chromatograms obtained according to one or more of the previous claims, wherein said integration provides for the identification of all peaks that have an area greater than 10 times the background noise of the chromatogram trace; using the peak of the reference compound as reference both for the quantitative analysis and to center the retention times,

where each peak is identified on the basis of:

- one signal m/z of quantization; and
- at least two signals m/z of qualification;
- quantification with the method of normalized percentages areas;
- transfer of the data obtained from said quantification to a matrix in which each sample represents a line and the columns are represented by various metabolites univocally identified by means of their chromatographic retention time.

**Patentansprüche**

1. Computergestütztes Verfahren zur Diagnose von Endometriumkarzinomen basierend auf der metabolomischen Analyse von Blut, wobei das Verfahren die folgenden Phasen umfasst:

   (I) eine Trainingsphase, umfassend:

   - GCMS- oder GCxGCMS-Analyse von Blutproben, die von Patienten mit einem Endometriumkarzinom und von gesunden Kontrollen stammen;
   - Integration der erhaltenen Ergebnisse durch eine multivariate Analyse unter Verwendung mindestens eines Diskriminanzanalysemodells oder eines Computerlernmodells zum Trainieren mindestens eines Klassifikationsmodells;

   (II) eine Zuordnungsphase, umfassend die GCMS- oder GCxGCMS-Analyse einer unbekannten Blutprobe und ihre Zuordnung zu einer Relevanzklasse auf der Basis des in der Trainingsphase (I) formulierten Klassifikationsmodells.

2. Verfahren nach Anspruch 1, wobei

   - mindestens ein Diskriminanzanalysemodell aus der Gruppe ausgewählt ist, die aus Folgendem besteht: PLS-DA und OPLS-DA, oder
   - wobei das Computerlernmodell aus der Gruppe ausgewählt ist, die aus Folgendem besteht: SVM und Entscheidungsbaum.

3. Verfahren nach einem oder mehreren der vorstehenden Ansprüche, wobei die Trainingsphase (I) die folgenden Unterphasen umfasst:

   - Extraktion und Derivatisierung von Metaboliten aus Blutproben, die von Patienten mit einem Karzinom und von gesunden Kontrollen stammen;
   - GCMS- oder GCxGCMS-Analyse von extrahierten und derivatisierten Metaboliten, um ein Chromatogramm für jede Probe zu erhalten;
   - Erstellung der Datenmatrix der metabolischen Profile der Patienten, die ein Endometriumkarzinom haben, und von gesunden Kontrollen;
   - Strukturieren mindestens eines Klassifikationsmodells als Ergebnis der multivariaten Datenfeldanalyse; wobei die multivariate Analyse unter Verwendung mindestens eines Diskriminanzanalysemodells oder eines Computerlernmodells zum Trainieren mindestens eines Klassifikationsmodells durchgeführt wird.

4. Verfahren nach einem oder mehreren der vorstehenden Ansprüche, wobei die Phase (II) weiter die folgenden Unterphasen umfasst:

   - Extraktion und Derivatisierung von Metaboliten von mindestens einer unbekannten Blutprobe;
   - GCMS- oder GCxGCMS-Analyse der extrahierten und derivatisierten Metaboliten, um mindestens ein Chromatogramm für die unbekannte Blutprobe zu erhalten;
   - Erstellung des metabolischen Profils aus dem Chromatogramm der unbekannten Blutprobe;
   - Zuordnung des metabolischen Profils zu einer Klasse auf der Basis des in Phase (I) trainierten Klassifikationsmodells.

5. Verfahren nach einem oder mehreren der vorstehenden Ansprüche, wobei die Anzahl von Blutproben, die von Patienten mit einem Endometriumkarzinom und von gesunden Kontrollen stammen, mindestens 80 % der Anzahl identifizierter Variablen metabolischer Profile entspricht.

6. Verfahren nach einem oder mehreren der vorstehenden Ansprüche, wobei das Klassifikationsmodell für eine dichotome Klassifizierung "Gesunder Patient" oder "Patient mit Endometriumkarzinom" trainiert wird.

7. Verfahren nach einem oder mehreren der vorstehenden Ansprüche, wobei das Klassifikationsmodell weiter für eine histologische Klassifikation von Krebs "Typ I" oder "Typ II" trainiert wird.

8. Verfahren nach einem oder mehreren der vorstehenden Ansprüche, wobei die Extraktion und Derivatisierung Folgendes umfassen:

   i) Rühren der aus der Zugabe einer Extraktionsmischung erhaltenen Probe;
   ii) Zentrifugieren der in i) erhaltenen Probe;
   iii) Derivatisierung des in ii) erhaltenen Überstands durch Behandlung mit Methoxyaminhydrochlorid in Pyridin;
   iv) Überstandsilanisierung der in iii) erhaltenen Probe mit einem Silanisierungsmittel, das aus der Gruppe ausgewählt ist, die aus Folgenden besteht: N,O-bis(Trimethylsilyl)trifluoracetamid (BSTFA), N-Methyl-N-(trimethylsilyl)trifluoracetamid (MSTFA), Hexamethyldisilazan (HMDS), l-(Trimethylsilyl)imidazol (TMSI), N-tert-Butyldimethylsilyl-N-methyltrifluoracetamid (MTBSTFA), l-(tert-Butyldimethylsilyl)imidazol (TBDMSIM);

   wobei die Extraktionsmischung aus einer wässrigen Mischung aus einem Alkohol und einem aprotischen polaren Lösungsmittel besteht.

9. Verfahren nach einem oder mehreren der vorstehenden Ansprüche, wobei die Extraktion von Metaboliten durch Hinzugeben einer Aliquote einer Referenzverbindung, vorzugsweise Ribitol, durchgeführt wird.

10. Verfahren nach einem oder mehreren der vorstehenden Ansprüche, weiter umfassend die folgenden Phasen:

   - Integration von Chromatogrammen, die nach einem oder mehreren der vorstehenden Ansprüche erhalten wurden, wobei die Integration die Identifizierung aller Spitzen, die eine Fläche aufweisen, die größer als das 10-Fache des Grundrauschens der Chromatogrammspur ist, vorsieht; unter Verwendung der Spitze der Referenzverbindung als Referenz sowohl für die quantitative Analyse als auch um die Retentionszeiten auszumitteln,

   wobei jede Spitze basierend auf Folgendem identifiziert wird:

   - ein Signal m/z der Quantisierung; und
   - mindestens zwei Signalen m/z der Qualifizierung;
   - Quantifizierung mit dem Verfahren der normalisierten Prozentflächen;
   - Übertragung der aus der Quantifizierung erhaltenen Daten auf eine Matrix, in der jede Probe eine Zeile darstellt und die Spalten durch verschiedene Metaboliten dargestellt werden, die mittels ihrer chromatographischen Retentionszeiten eindeutig identifiziert wurden.

**Revendications**

1. Procédé assisté par ordinateur pour le diagnostic du carcinome de l'endomètre sur la base d'une analyse de sang métabolomique, ledit procédé comprenant les phases suivantes :

   (I) une phase d'apprentissage comprenant :

   - l'analyse par GC-MS ou GCxGC-MS d'échantillons sanguins dérivés de patientes atteintes de carcinome de l'endomètre et de témoins en bonne santé ;
   - l'intégration des résultats obtenus par une analyse multivariée utilisant au moins un modèle d'analyse discriminante ou un modèle d'apprentissage informatique pour former au moins un modèle de classification ;

   (II) une phase d'affectation comprenant l'analyse GC-MS ou GCxGC-MS d'un échantillon sanguin inconnu et son affectation à une classe pertinente sur la base du modèle de classification formulé lors la phase d'apprentissage (I).

2. Procédé selon la revendication 1 dans lequel

- au moins un modèle d'analyse discriminante est sélectionné dans le groupe constitué par : le PLS-DA et l'OPLS-DA, ou
- ledit modèle d'apprentissage informatique est sélectionné dans le groupe constitué par : la SVM ou l'arbre de décision.

3. Procédé selon l'une quelconque des revendications précédentes, dans lequel la phase d'apprentissage (I) comprend les sous-phases suivantes :

- l'extraction et la dérivation à partir de métabolites d'échantillons sanguins dérivés de patientes atteintes de carcinome et de témoins en bonne santé ;
- l'analyse par GC-MS ou GCxGC-MS de métabolites extraits et dérivés pour obtenir un chromatogramme pour chaque échantillon ;
- la création d'une matrice de données des profils métaboliques des patientes atteintes de carcinome de l'endomètre et de témoins en bonne santé ;
- la structuration d'au moins un modèle de classification suite à l'analyse multivariée de réseau de données ; dans lequel ladite analyse multivariée est réalisée en utilisant au moins un modèle d'analyse discriminante ou un modèle d'apprentissage informatique pour former au moins un modèle de classification.

4. Procédé selon une ou plusieurs des revendications précédentes, dans lequel ladite phase (II) comprend en outre les sous-phases suivantes :

- l'extraction et la dérivation de métabolites d'au moins un échantillon sanguin inconnu ;
- l'analyse par GC-MS ou GCxGC-MS des métabolites extraits et dérivés pour obtenir au moins un chromatogramme pour l'échantillon sanguin inconnu ;
- la création d'un profil métabolique à partir dudit chromatogramme dudit échantillon sanguin inconnu ;
- l'affectation du profil métabolique à une classe sur la base du modèle de classification appris dans la phase (I).

5. Procédé selon une ou plusieurs des revendications précédentes dans lequel le nombre d'échantillons sanguins dérivés de patientes atteintes de carcinome de l'endomètre et de témoins en bonne santé est égal à au moins 80 % du nombre de variable identifiées de profils métaboliques.

6. Procédé selon une ou plusieurs des revendications précédentes dans lequel ledit modèle de classification est appris pour une classification dichotomique « Patiente en bonne santé » ou « Patiente affectée par le carcinome de l'endomètre ».

7. Procédé selon une ou plusieurs des revendications précédentes dans lequel ledit modèle de classification est appris en outre pour une classification histologique d'un cancer de « type 1 » ou de « type II ».

8. Procédé selon une ou plusieurs des revendications précédentes dans lequel lesdites extraction et dérivation comprennent :

i) l'agitation de l'échantillon obtenu à partir de l'ajout d'un mélange d'extraction ;
ii) la centrifugation de l'échantillon obtenu dans i) ;
iii) la dérivation du surnageant obtenu dans ii) par traitement avec du chlorhydrate de méthoxyamine dans de la pyridine ;
iv) la silanisation du surnageant de l'échantillon obtenu dans iii) avec un agent de silanisation sélectionné dans le groupe constitué par : le N,Obis(triméthylsilyl)trifluoroacétamide (BSTFA), le N-méthyl-N-(triméthylsilyl)trifluoroacétamide (MSTFA), l'hexaméthyldisilazane (HMDS), le 1-(triméthylsilyl)imidazole (TMSI), le N-tert-butyldiméthylsylyl-N- méthyltrifluoroacétamide (MTBSTFA), le 1-(tert-butyldiméthylsilyl)imidazole (TBDMSIM) ;

dans lequel ledit mélange d'extraction est constitué par un mélange aqueux d'un alcool et d'un solvant polaire aprotique.

9. Procédé selon une ou plusieurs des revendications précédentes dans lequel ladite extraction de métabolites est réalisée en ajoutant une aliquote d'un composé de référence, préférablement de ribitol.

10. Procédé selon une ou plusieurs des revendications précédentes comprenant les phases suivantes :

- l'intégration de chromatogrammes obtenus selon l'une quelconque des revendications précédentes, dans lequel ladite intégration fournit l'identification de tous les pics qui ont une aire supérieure à 10 fois le bruit de fond de la trace de chromatogramme ; en utilisant le pic du composé de référence comme référence à la fois pour l'analyse quantitative et pour centrer les temps de rétention,

où chaque pic est identifié sur la base :

- d'un signal m/z de quantification ; et
- d'au moins deux signaux m/z de qualification ;
- de quantification avec le procédé des aires de pourcentages normalisés ;
- de transfert des données obtenues à partir de ladite quantification vers une matrice dans laquelle chaque échantillon représente une ligne et les colonnes sont représentées par divers métabolites identifiés de manière univoque au moyen de leur temps de rétention chromato graphique.

OPLS-DA Classification

Histotype Classification

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007057309 B
- WO 2011161186 A
- JP 2007147459 A

### Non-patent literature cited in the description

- **MULLER GL et al.** Allelotype mapping of unstable microsatellites establishes direct lineage continuity between endometrial precancers and cancers. *Cancers Res,* 1996, vol. 56, 4483
- **KADDURAH-DAOUK R.** Metabolic profiling of patients with schizophrenia. *PLOS Med,* 2006, vol. 8, e363
- **SUBRAMANIAN A. et al.** Proton MR/CSF analysis and a new software as predictors for the differentiation of meningitis in children. *NMR Biomed,* 2005, vol. 18, 213-25
- **DENKERT C. et al.** Metabolite profiling of human colon carcinoma - deregulation of TCA cycle and amino acid turnover. *Mol. Cancer,* 2008, vol. 7, 1-15
- **FAN L. et al.** Identification of metabolic biomarkers to diagnose epithelial ovarian cancer using a UPLC/QTOF/MS platform. *Acta Oncologica,* 2012, vol. 51, 473-479
- **VAPNIK, V.** Estimation of Dependences Based on Empirical Data. Springer Verlag, 1982
- **KARKKAINEN J et al.** *EUROPEAN JOURNAL OF CANCER AND CLINICAL ONCOLOGY,* 1990, vol. 26 (9), 975-977
- **CAO Q JACKIE et al.** *International Journal of Gynecological Pathology,* vol. 23 (4), 321-329
- **PETER PODOLEC et al.** *JOURNAL OF CHROMATOGRAPHY B: BIOMEDICAL SCIENCES & APPLICATIONS,* 2014, vol. 967, 134-138